# FASCICULE DE BREVET EUROPEEN

(11) **EP 0 955 069 B1**
(45) Date de publication et mention de la délivrance du brevet: **02.08.2006**
(21) Numéro de dépôt: 99490013.2
(22) Date de dépôt: 30.04.1999
(51) Int. Cl.: A61L 31/16, D06M 16/00

(54) **Procédé pour conférer à un matériau des propriétés biocides et/ou biostatique stables**
Verfahren zur Vermittlung von bioziden und/oder biostatischen Eigenschaften an ein Material
Process for imparting biocidal and/or biostatic properties to a material

(30) Priorité: 30.04.1998 FR 9805756
(43) Date de publication de la demande: 10.11.1999
(73) Titulaire: INSTITUT FRANCAIS DU TEXTILE ET DE L'HABILLEMENT, 69134 Ecully Cedex (FR)
(72) Inventeur: Chatelin, Roger Jean Robert, Domaine de Bois Dieu, 69380 Lozanne (FR); Bourgeois, Michel, 69003 Lyon (FR)
(74) Mandataire: Hennion, Jean-Claude

(56) Documents cités:
- WO-A-95/12021
- US-A- 3 898 336
- US-A- 5 141 803

## Description

La présente invention concerne un matériau, en particulier un matériau à grande surface spécifique, notamment fibreux, de type textile, poreux ou alvéolaire souple ou rigide présentant des propriétés biocides et/ou biostatiques stables à l'entretien domestique. Elle concerne plus précisément un procédé de traitement d'un matériau permettant de conférer à celui-ci lesdites propriétés.

La mise en oeuvre d'agents antiseptiques, pour traiter des matériaux textiles et conférer à ceux-ci des propriétés antiseptiques ou biocides est déjà bien connue. Parmi les agents antiseptiques et désinfectants potentiellement létaux, on distingue les composés chimiquement très actifs qui se caractérisent par une action brutale, rapide temporaire et souvent non spécifique et des composés chimiquement stables à action plus spécifique. Parmi les premiers composés, on trouve les oxydants, l'oxyde d'éthylène, les acides forts et les bases fortes, les aldéhydes et le phénol. Parmi les seconds composés, on trouve notamment les ammoniums quaternaires, les dérivés phénoliques, la chlorhexidine et les polyhexaméthylènebiguanides (PHMB).

Il a déjà été proposé dans le document EP 0 136 900 de traiter les matériaux textiles avec des PHMB afin de conférer à ceux-ci des propriétés anti-microbiennes ou antiseptiques. Les biguanides présentent une bonne substantivité pour les matériaux cellulosiques purs ou en mélange. Cependant, beaucoup de détergents disponibles sur le marché contiennent des agents de blanchiment chlorés qui réagissent avec les groupes biguanides, ce qui a pour effet de provoquer un jaunissement du matériau textile et de réduire la protection anti-microbienne. De plus, beaucoup de détergents sur le marché contiennent des surfactants anioniques qui forment des complexes avec les groupes biguanides, ce qui a pour effet de donner au matériau un toucher poisseux et d'altérer les propriétés d'entretien du matériau.

On a déjà proposé dans le document WO 95/12021 de remédier à ces inconvénients. Selon ce document antérieur, le procédé de traitement du matériau textile consiste à appliquer sur celui-ci d'une part un oligomère ou polymère de biguanide ou un sel de celui-ci avec un acide inorganique ou un acide organique ayant une valeur de pK supérieure à 4,5 et d'autre part un acide organique fort ayant une valeur de pK inférieure à 4,5 et exempt de toute chaîne aliphatique ou oxyalkylène contenant douze atomes de carbone ou plus.

Selon ce document c'est la présence de l'acide organique fort qui empêche les effets négatifs dus à la présence de certains détergents lors de l'entretien du textile.

Cependant, même si le traitement préconisé par le document WO 95/12021 est susceptible d'apporter une solution pour les textiles cellulosiques à l'égard desquels les biguanides ont une bonne substantivité, cette solution n'est pas totalement satisfaisante puisqu'elle n'est pas transposable en l'état aux autres matériaux textiles. De plus, même s'agissant de textiles cellulosiques, selon le demandeur les propriétés antiseptiques n'ont pas une durabilité suffisante ni une résistance suffisante aux conditions d'usage et d'entretien domestique, y compris le nettoyage à sec.

Le but que s'est fixé le demandeur est de proposer un procédé de traitement plus spécialement adapté aux matériaux à grande surface spécifique, notamment fibreux ou poreux, en vue de leur conférer des propriétés biocides et/ou biostatiques stables à l'entretien domestique.

Ce but est parfaitement atteint par un procédé de traitement d'un matériau qui, de manière caractéristique met en oeuvre une solution de traitement dans laquelle est dissoute une molécule ionique et/ou un polymère ionique et qui comprend une ou plusieurs étapes au cours de laquelle ou desquelles, d'une part on applique sur le matériau ladite solution de traitement et d'autre part la molécule ionique et/ou le polymère ionique réagit avec un agent précipitant pour former in situ, sur le matériau un précipité insoluble présentant des propriétés biocides et/ou biostatiques ; ladite molécule ionique et/ou ledit polymère ionique et/ou l'agent précipitant étant biocide(s) et/ou biostatique(s) .

On définit le caractère biocide comme la propriété de détruire des microorganismes, le caractère biostatique étant défini comme la limitation de la prolifération des microorganismes.

On définit, dans le cadre de la présente invention, un polymère comme étant une molécule ionique obtenue par polymérisation, et ayant par exemple une masse molaire moléculaire supérieure à 2000. On définit au contraire une molécule ionique comme étant une molécule de taille plus réduite, par exemple, ayant une masse inférieure à 2000 et qui est donc susceptible de mieux diffuser en particulier dans un matériau fibreux, poreux ou présentant une surface complexe.

L'agent précipitant peut être, dans le cadre de la présente invention, soit une molécule ionique, soit un polymère ionique soit le matériau à traiter lui-même, lorsqu'une réaction entre la surface du matériau et la molécule ou le polymère ionique contenu dans la solution de traitement est possible.

L'application de la solution de traitement peut être réalisée par imprégnation, pulvérisation ou toute autre méthode connue de dispersion d'un liquide.

On définit par stable un matériau qui présente des propriétés biocides et/ou biostatiques qui persistent après au moins cinq lavages dans des conditions d'entretien normalisées.

La formation d'un précipité pour conférer à un matériau des propriétés biocides a déjà été utilisée dans le document US 3 898 336. Néanmoins, le procédé décrit dans ce document ne concerne d'une part que le traitement de matériaux qui ne sont pas susceptibles ou destinés à être lavés (fil de suture ou bandages) et d'autre part il ne permet pas d'obtenir des propriétés biocides stables dans le temps et surtout résistantes au lavage.

En effet, selon ce document, on dissout dans un solvant approprié un polymère linéaire ammonium quaternaire ne présentant pas lui-même de propriétés biocides et/ou biostatiques , du fait de la présence d'un seul atome halogène au voisinage du cation N⁺; on imprègne ensuite le matériau à traiter du polymère dissous et l'on ajoute un générateur d'ions halogène, à savoir brome et iode, en vue de précipiter sur le matériau un sel insoluble trihalogéné du polymère qui, du fait de la présence de trois atomes d'halogène au voisinage du cation N⁺ , présente des propriétés biocides par relarguage des halogènes. Or, les polymères trihalogénés obtenus ont d'une part un caractère instable qui limite dans le temps l'efficacité biocide du matériau sur lequel ils sont déposés, même sans lavage de celui-ci. D'autre part, les ions halogènes qui confèrent les propriétés biocides étant de petite taille, ils sont rapidement éliminés lors du lavage du matériau qui perd donc rapidement ses propriétés. De plus, le caractère toxique de ces polymères trihalognés rend le matériau impropre, en particulier, à un usage domestique, du fait du danger qu'il est susceptible de représenter pour l'utilisateur et son entourage. Il était donc à priori impossible d'obtenir par précipité un matériau présentant des propriétés biocides et/ou biostatiques susceptibles d'être stables au lavage.

Selon un premier mode de réalisation de l'invention, après application de la solution de traitement sur le matériau et son éventuel séchage, on utilise comme agent précipitant une solution ou une émulsion contenant au moins une molécule ionique et/ou au moins un polymère ionique en sorte d'obtenir un précipité insoluble.

Le séchage présente l'avantage de mieux confiner le produit à précipiter sur ou dans une structure fibreuse ou poreuse. Cependant il peut être procédé sans séchage, par imprégnation-essorage ou encore à l'aide d'un bain court de type pulvérisation ou léchage.

Ainsi, dans ce mode de réalisation, on met à profit ce qui dans le document antérieur WO 95/12021 était considéré comme un inconvénient majeur à savoir la précipitation du polymère. Dans le cas présent, cette précipitation intervient de manière à l'immobiliser in situ, en particulier dans un matériau à grande surface spécifique, notamment fibreux ou poreux, en sorte d'empêcher ou de limiter considérablement son extraction au cours des cycles d'entretien.

Le choix de l'utilisation d'un polymère ou d'une molécule biocide et/ou biostatique pour la solution de traitement permet d'obtenir des matériaux présentant des propriétés durant un temps relativement long ; en effet, la molécule ou le polymère bioactif (biocide et/ou biostatique) étant au contact du matériau, le précipité formé plus en surface du matériau protège en quelque sorte la molécule ou le polymère bioactif en prévenant notamment son lessivage.

De même l'utilisation d'un agent précipitant bioactif permet d'obtenir des matériaux ayant une forte activité biocide et/ou biostatique, puisque la molécule ou le polymère bioactif est dans ce cas disposé plus à la surface du matériau.

La présence de molécules ioniques dans la solution de traitement permet, du fait de la faible taille de ces molécules, de les disperser par diffusion uniformément sur ou dans le matériau ce qui s'avère particulièrement intéressant lorsque le matériau est fibreux ou poreux. La dispersion uniforme par diffusion de ces molécules ioniques permet une répartition et une accroche uniforme du précipité formé sur le matériau.

De même, la présence de polymère(s) dans la solution de traitement peut s'avérer intéressante car les molécules de polymère étant relativement longues, elles peuvent s'accrocher à la surface du matériau en particulier lorsque celui présente une surface irrégulière tel qu'une moquette, par exemple, et garantir ainsi l'accroche mécanique du précipité formé.

La solution de traitement peut être appliquée sur le matériau par immersion ou imprégnation du matériau, pulvérisation ou toute autre méthode connue permettant de disperser une phase liquide.

L'utilisation d'un mélange de polymères et/ou de molécules ioniques pour la solution de traitement et/ou pour l'agent précipitant permet d'élargir le spectre d'action du matériau traité. Par exemple, dans le cas des polymères, ceux-ci présentant plusieurs sites qui sont susceptibles de réagir chacun avec une sorte de polymère ou une sorte de molécule ionique, il est ainsi possible de faire réagir un polymère avec plusieurs types de molécules ou de polymères biocides et/ou biostatiques ayant des spectres d'action différents voire complémentaires.

Selon une première variante, la solution de traitement ou l'agent précipitant contient une solution aqueuse contenant 5% de chlorure de benzalkonium et 5% de docécylsulfate de sodium.

Selon une seconde variante, la solution de traitement ou l'agent précipitant contient un polymère cationique biocide choisi parmi le groupe formé par les polymères polyhexaméthylène biguanides bactéricides (PHMB) et par les polymères ammonium quaternaire bactéricides.

L'utilisation d'un polymère biocide et/ou biostatique qui de par la taille de ses molécules n'est pas lessivable du fait de son accrochage à la surface du matériau, permet de garantir au matériau traité par le procédé de la présente invention, des propriétés biocides et/ou biostatiques résistantes au lavage.

On sait que pour être solubles dans l'eau, les polyhexaméthylène biguanides sont généralement présentés sous forme de chlorhydrates ou sous formes de sels d'acide minéral fort tels que sulfates ou nitrates ou encore sous forme de sels d'acides faibles tels que acétates, propionates ou gluconates.

Selon une troisième variante, la solution de traitement ou l'agent précipitant contient un anion silicate.

Selon une quatrième variante, la solution de traitement ou l'agent précipitant contient un polymère anionique alkylé ayant au moins dix atomes de carbone, par exemple un savon de sodium de C12 à C22, notamment oléate de sodium, alkylbenzène sulfonate de sodium ou tripolyphosphate de sodium, utilisée en tant qu'agent précipitant.

Ces polymères anioniques alkylés sont des agents tensio-actifs anioniques lourds que l'on rencontre dans la composition de certaines lessives et qui donnent des précipités en particulier avec les polymères ammonium quaternaire. Il est à remarquer que ces produits anioniques ont une aptitude à réagir avec les polymères cationiques, notamment ammonium quaternaire, ladite aptitude étant supérieure à celle des autres constituants lessiviels et à celle des anions à faible masse molaire généralement utilisés. L'affinité en échange d'ion croît avec la masse molaire et la valence des ions mis en jeu. De plus, l'insolubilité et l'hydrophobie du sel issu de la réaction "anion + cation" seront d'autant plus fortes que le caractère ionique résiduel sera faible, et que la masse molaire du couple anion-cation obtenu sera élevée.

Ainsi, de manière remarquable, selon le procédé de l'invention, l'immobilisation du polymère in situ dans le matériau ne peut qu'être accentué par des cycles d'entretien mettant en oeuvre des lessives de ce type.

Selon une cinquième variante, la solution de traitement ou l'agent précipitant contient une solution de dodécyl sulfate de sodium qui est lui-même un agent tensio-actif biocide et qui permet donc, en association avec une molécule ionique ou un polymère ionique biocide d'élargir le spectre d'action du matériau traité par le procédé de l'invention.

Dans un second mode de réalisation, le procédé de l'invention comporte une étape consistant à greffer sur le matériau des greffons porteurs de fonctions anioniques ou cationiques qui ont la fonction d'agent précipitant et qui sont donc aptes à précipiter la molécule et/ou le polymère ionique dissout(e)s dans la solution de traitement.

Au cours de l'étape d'application de la solution de traitement, le contre ion du polymère ionique soluble ou de la molécule ionique de la solution de traitement est déplacé par la fonction anionique ou cationique portée par les greffons et de ce fait il y a formation d'un précipité lié aux greffons du matériau, en conservant les caractéristiques d'accessibilité et de mobilité connues et mises à profit dans le cas d'un greffage.

L'immobilisation, dans ce second mode de réalisation, intervient grâce aux forces de liaison entre la partie cationique ou anionique du polymère et la fonction anionique ou cationique portée par les greffons, eux-mêmes fixés par liaison covalente au matériau.

De préférence, on met en oeuvre des acrylates ou méthacrylates, porteurs de fonctions anioniques.

De préférence, s'agissant d'une fonction carboxylique ou carboxylate, on utilise comme monomère l'acide acrylique ou l'acide méthacrylique.

De préférence, s'agissant d'une fonction sulfonique ou sulfonate, on utilise comme monomère l'acide 2-acrylamido-2-méthylpropane sulfonique (AMPS).

De préférence, s'agissant d'une fonction phosphorique ou phosphate, on utilise comme monomère le phosphate de méthylacrylate éthylène glycol (PMEG).

Le procédé de la présente invention s'applique plus particulièrement à un matériau poreux ou fibreux qui présente donc une grande surface spécifique.

La présente invention sera bien comprise à la lecture de la description qui va être faite de plusieurs exemples de réalisation d'un matériau fibreux ou poreux traité en vue de lui conférer des propriétés antiseptiques durables et stables à l'entretien, que cet entretien soit par des cycles de lavage lessiviel ou par entretien à sec.

Conformément à l'invention, le procédé utilisé met en oeuvre un polymère cationique antiseptique, choisi parmi le groupe formé par les polymères polyhexaméthylène biguanides (PHMB) et les polymères ammonium quaternaire.

Le matériau à grande surface spécifique peut se présenter sous forme d'un tissu, d'un tricot tubulaire, d'un tricot plat, d'un non-tissé, d'un câble ou d'un fil de filaments continus, d'un filé de fibres éventuellement creuses ou microporeuses, d'une mousse, d'une éponge, d'un papier, d'un carton, de bois, de céramique, ou de tout autre matériau apparenté.

Les PHMB sont des polymères cationiques antiseptiques dont la structure générale est la suivante : 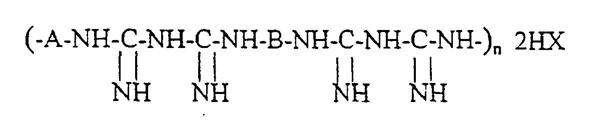 dans laquelle :
n ≥4
X est un anion monovalent inorganique de faible masse molaire (halogénure, sulfate, ...).
A et B sont des groupes d'atomes qui peuvent être identiques ou différents.
Le composé PHMB préféré est connu sous la dénomination VANTOCIL IB ® de la firme ZENECA, qui a la formule suivante : 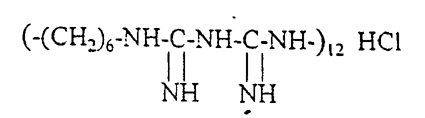 Les polymères cationiques ammonium quaternaire antiseptiques comportent les groupements suivants : 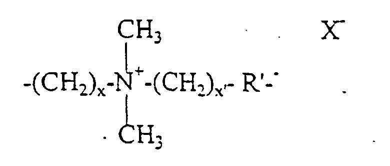 dans lesquels x et x' ≥ 1
X est un anion monovalent inorganique de faible masse molaire (halogénure, sulfate , ...).
ou 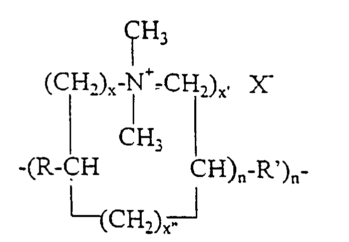 dans lesquels x ou x' ≥ 1, x" ≥ 0
n>4
R, R' sont des groupes d'atomes identiques ou différents, pouvant comporter des hétéro-atomes.
X est de préférence Cl⁻ ou un anion monovalent inorganique de faible masse molaire (halogénure, sulfate, carbonate, ...).
Un polymère ammonium quaternaire et cationique à propriété antiseptique préféré est connu sous la dénomination MIRAPOL®A 15 de RHONE POULENC ayant la formule suivante : 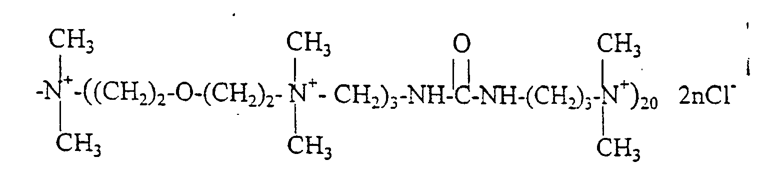 avec n = 20.
Un autre polymère cationique préféré est connu, sous la dénomination RHOQUAT®JL de la firme RONDOT ayant la formule suivante : 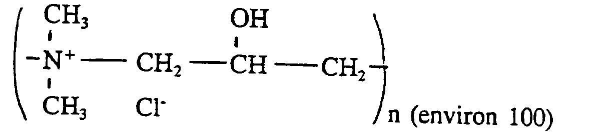 Il est aussi possible de mettre en oeuvre des polymères ammonium quaternaire issus de la polymérisation de monomères du type acrylate ou méthacrylate d'ammonium quaternaire, par exemple ayant la formule générale suivante : 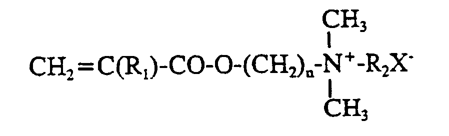 dans laquelle :
n : supérieur ou égal à 1
R₁ : H ou CH₃
R₂: chaîne carbonée de plus de 6 carbones
X : anion monovalent inorganique de faible masse molaire (halogénure, sulfate, etc...).

Les PHMB comme les polymères ammonium quaternaire sont très solubles dans l'eau et l'alcool.

Le mécanisme biocide des PHMB, leur conférant leurs propriétés antiseptiques, est une action sur les membranes externes des cellules suivantes. Ils ont un large spectre d'activité sur l'ensemble des bactéries, leur activité étant plus faible vis-à-vis des champignons et des levures. Ils sont connus dans leur utilisation comme conservateurs dans les collyres et les cosmétiques et comme désinfectants et sont donc totalement inoffensifs ce qui permet un usage domestique du matériau traité par le procédé de l'invention.

Le mode d'action des polymères ammonium quaternaire est l'abaissement de la tension superficielle aux interfaces des cellules de bactéries, de levures et de moisissures. Les cellules chargées négativement sont neutralisées, ce qui entraîne des changements de perméabilité et des lésions de la membrane cytoplasmique et de la paroi. Le contenu cellulaire est libéré dans le milieu environnant. Il y a inactivation des enzymes respiratoires, dénaturation et précipitation des protéines et des acides nucléiques. Les polymères ammonium quaternaire possèdent une certaine activité sur les parasites comme les amibes et les trypanosomes. Certains ont une activité anti-algue.

Les deux familles de produits, que sont les PHMB et les polymères ammonium quaternaire cationiques possèdent des spectres complémentaires pour la plupart des germes usuels. Avantageusement, on met en oeuvre un mélange de ces deux produits.

De manière caractéristique, selon l'invention, on réalise l'insolubilisation in situ dans le matériau du PHMB ou du polymère ammonium quaternaire, cette insolubilisation étant réalisée par le déplacement de l'anion de faible masse molaire.
Dans une première variante de réalisation, cette insolubilisation est obtenue en déplaçant l'anion du polymère antiseptique cationique par un anion à haut poids moléculaire de sorte que le sel obtenu est insoluble ou peu soluble. Si l'un des deux réactifs, polymère cationique antiseptique ou anion lourd, est placé préalablement sur le matériau fibreux ou poreux et que l'on ajoute le second réactif , on immobilise le sel obtenu sur le support.

Le placement préalable sur le matériau fibreux ou poreux peut être obtenu par imprégnation éventuellement suivie d'un séchage. Le séchage présente l'avantage de mieux confiner le produit à précipiter sur ou dans la structure fibreuse ou poreuse. L'opération peut néanmoins ête réalisée sans séchage, à l'aide d'un bain court de type pulvérisation ou léchage, ce qui élimine une opération. L'addition du second réactif peut être obtenue par pulvérisation ou imprégnation. L'anion lourd dont il s'agit peut être un mono-anion, un polyanion ou un anion complexe, par exemple, un mono-anion de formule CₙH₂ₙ₊₁-A⁻ (n supérieur ou égal à 12), par exemple un polyanion de formule 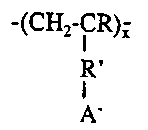 dans laquelle :
x supérieur à 10
R est H ou CH₃
R' est un groupe d'atomes,
par exemple un anion complexe minéral, de type silicate, polyphosphate...

Le demandeur a tout d'abord vérifié le caractère insoluble du précipité formé en ajoutant à une solution aqueuse à 10 % en PHMB sous forme de chlorhydrate et sous agitation de différentes solutions, à savoir une solution d'oléate de sodium à 10 %, une solution de dodécyl sulfate de sodium à 10 % et une solution de lessive ECE à 10 g/l. Dans les trois cas un précipité blanc s'est formé, que l'on a laissé décanter plusieurs heures. Ce précipité a été filtré, rincé à l'eau et séché ; il s'est avéré insoluble dans l'eau, l'alcool et la soude.

La solution de lessive ECE à 10 g/l est une lessive normalisée, utilisée dans la norme d'entretien lessiviel sous la référence NF EN 26330. Cette lessive contient notamment à raison de 8 % en matière sèche de l'alkyl benzène sulfonate de sodium dans lequel de la chaîne alkyle comporte en moyenne 11,5 atomes de carbone et à raison de 3,5 % en matière sèche du savon de sodium dont la chaîne alkyle comporte de 12 à 22 atomes de carbone.

Le demandeur a dans un deuxième temps vérifié que le précipité insoluble obtenu à partir de la solution de PHMB était bien immobilisé in situ dans un matériau fibreux ou poreux avec une étape d'imprégnation et une étape d'insolubilisation.

Pour cela, il a préparé une solution à 10 % en PHMB sous forme de chlorydrate dans l'eau, puis imprégné par foulardage-exprimage un tissu de coton d'armure toile, tissé avec des fils de Nm 50, d'un grammage de 160 g/m² et préalablement blanchi. Il a ensuite pulvérisé sur des échantillons de ce tissu les solutions respectivement d'oléate de sodium, de dodécyl sulfate de sodium et de lessive ECE précitées. Les échantillons ont été séchés et pesés de manière à mesurer le gain de poids obtenu, de l'ordre de 9 ou 10 %. Les échantillons ont été rincés en machine à laver, à l'eau froide et de nouveau séchés puis pesés. Le gain de poids final obtenu est de l'ordre de 8-9 %. Ce gain de poids correspond au polymère antiseptique précipité in situ dans le tissu.

Le demandeur a enfin vérifié dans un troisième temps l'existence et la permanence des propriétés antiseptiques du tissu ainsi traité, après cinq lavages en machine à laver à 40 °C avec une solution de lessive ECE à 3 g/l. Cette vérification a été réalisée en utilisant un test microbiologique qualitatif selon la norme suisse SNV 195 920 pour la détermination de l'effet anti-bactérien, en mettant en oeuvre comme souche bactérienne le Staphylococcus aureus ATCC 9144.

Le résultat de ce test est une cotation 0 avec une zone d'inhibition (ZI) de 0 à 1 mm. Ainsi, après cinq lavages en machine à laver, le tissu traité présente de bonnes propriétés antiseptiques contre la flore bactérienne avec une zone d'inhibition très limitée voire nulle. En d'autres termes, les propriétés biocides sont limitées au tissu proprement dit, sans diffusion vers l'extérieur. C'est ce qui est recherché en l'occurence. Les mêmes résultats ont été obtenus pour les trois échantillons de tissu traité.

Ainsi, a pu être vérifé, que le polymère antiseptique cationique PHMB conservait ses propriétés antiseptiques malgré la présence de l'anion lourd et malgré son immobilisation in situ dans le matériau fibreux.

Cette vérification a été également faite en utilisant comme polymère antiseptique ammonium quaternaire le MIRAPOL®A 15 et également le MIRAPOL®550 dont la formule diffère du MIRAPOL® A 15 en ce qu'il s'agit d'un copolymère où la fonction ammonium quaternaire est intégrée dans un cycle pipéridinium, en copolymérisation avec facrylamide.

A partir d'une solution à 10 % de MIRAPOL®on a imprégné un tissu coton du type toile faisant 160 g/m² par foulardage exprimage. Après séchage, on a mesuré le gain de poids obtenu qui était de 7 %. Après cinq lavages en machine à laver à 40 ° C avec une solution de lessive ECE à 3 g/l, le tissu a été testé selon la norme SNV 195 920 en mettant en oeuvre comme souches bactériennes le Staphylococcus aureus ATCC 9144 et également le Pseudomonas aeruginosa CIP A 22. Pour ces deux souches, les tissus traités à partir du MIRAPOL®A 15 et du MIRAPOL®550 ont donné les même résultats, à savoir une cotation 0 et une zone d'inhibition de 0 à 1 mm.

On a de plus vérifié l'existence et la permanence des propriétés antiseptiques en utilisant un test microbiologique qualitatif selon la norme suisse SNV 195 921 pour la détermination de l'effet antifongique, en mettant en oeuvre comme souches bactériennes Candida albicans ATCC 10 259, Aspergillus niger et Trichophyton interdigitale CBS 42 863. Les résultats ont été, pour ces trois dernières souches, une cotation 0 et une zone d'inhibition respectivement de 1 à 2 mm, pour la première et de 0 à 1 mm pour la deuxième et la troisième.

Ces tests microbiologiques consistent à déposer des éprouvettes de support traitées et non traitées (témoins) sur une gélose d'une boîte de Pétri ensemencée avec une suspension de bactéries, de levures ou de spores de champignons, de la concentration approximative 10⁵ UFC/ml (UFC signifiant Unité Formant Colonies). Après 24 h, on constate si les éprouvettes sont propres ou contaminées, avec ou sans zone d'inhibition. On appelle zone d'inhibition (ZI) toute zone autour de l'échantillon de support exempte de colonies. L'efficacité anti-microbienne de l'éprouvette est évaluée d'après l'observation des contaminations suivant le système de cotation suivant :
cotation 0 : pas de contamination, avec ou sans zone d'inhibition,
cotation 1 : contamination faible, pas de colonies,
cotation 2 : contamination importante, présence de nombreuses colonies.

Dans une seconde variante de réalisation, l'insolubilisation in situ dans le matériau fibreux ou poreux du PHMB ou du polymère ammonium quaternaire antiseptique est obtenu en déplaçant l'anion du polymère antiseptique cationique par un anion qui est lui-même porté par un greffon fixé sur le matériau fibreux ou poreux. Conformément à l'invention, on réalise tout d'abord le greffage du matériau fibreux ou poreux en mettant en oeuvre au moins un monomère comportant au moins une fonction anionique. Pour réaliser ce greffage, il faut introduire sur le matériau à traiter des radicaux capables de réagir avec le ou les monomères. A partir de ces radicaux se développent des chaînes polymériques latérales, dénommées greffons.

Dans les exemples de réalisation ci-dessous, l'activation qui génère les radicaux capables de réagir avec le ou les monomères a été réalisée par irradiation simultanée. Le matériau fibreux ou poreux a tout d'abord été imprégné avec la solution de monomère choisie puis irradiée sous faisceaux d'électrons, de préférence avec un inertage sous atmosphère d'azote. Les sites radicalaires sont créés, du fait de l'irradiation, à la fois sur le matériau et sur le monomère. Il est également possible de faire une pré-irradiation du matériau, puis une imprégnation du matériau irradié avec la solution du greffage.

### Exemple 1

Dans un premier exemple de réalisation, on a préparé une solution aqueuse à 10 % d'acide acrylique. On a imprégné un tissu coton du type toile de 160 g/m² par foulardage sxprimage avec un taux d'imprégnation de 75 %. On a soumis ce tissu à une irradiation par faisceaux d'électrons à 10 kGy, l'énergie des électrons étant de 430 keV, le tissu a été rincé puis séché et pesé. On a pu constater un taux de greffage de l'ordre de 2 à 7 %. Ce tissu greffé a été mis en contact avec une solution de carbonate de sodium à 5 %, puis rincé et séché. Il a ensuite été mis en contact avec une solution aqueuse à 5 % de PHMB sous forme chlorydrate. Il a encore été rincé et séché. Sur ce tissu, on a effectué un test microbiologique selon la norme SNV 195 920 avec comme souche Staphylococcus aureus. On a obtenu une cotation 0 avec une zone d'inhibition de 7 à 8 mm. On a ensuite procédé à cinq lavages en machine à laver à 40° C avec une solution de lessive ECE à 3 g/l puis on a procédé au même test selon la norme SNV 195 920. Avec la souche Staphylococcus aureus, on a obtenu une cotation de 0 et une zone d'inhibition de 1 à 3 mm. Ainsi, les cinq lavages n'ont pas altéré les propriétés antiseptiques conférées par le traitement de l'invention ; ils ont simplement diminué la zone d'inhibition sur gélose, ce qui est favorable pour les applications recherchées..

Avec la souche Pseudomonas aeruginosa, le même tissu traité comme ci-dessus, après cinq lavages, avait une cotation de 0 et une zone d'inhibition de 1 à 2 mm. On a procédé à des tests microbiologiques selon la norme SNV 195 921, avec le même tissu traité et après cinq lavages, les résultats étaient dans tous les cas une cotation 0 et une zone d'inhibition respectivement de 2 à 3 mm avec la souche Candida albicans, 0 à 1 mm avec les souches Aspergillus niger et Tricophyton interdigitale.

### Exemple 2

Le même essai a été réalisé en mettant en oeuvre non plus une solution aqueuse d'acide acrylique mais une solution aqueuse à 40 % en AMPS (acide-2- acrylamido-2-methylpropane sulfonique). Les tests selon la norme SNV 195 920 ont donné, avant tout cycle d'entretien, avec la souche Staphylococcus aureus une cotation 0 et une zone d'inhibition de 6 à 7 mm. Après cinq cycles de nettoyage à sec selon la norme ISO 3175 Cycle II "articles sensibles", le tissu a été de nouveau testé selon la norme SNV 195 920, ce qui a donné comme résultats une cotation 0 et une zone d'inhibition de 1 à 2 mm avec la souche Staphylococcus aureus, de même qu'avec la souche Pseudomonas aeruginosa. Les mêmes résultats qu'avec l'acide acrylique ont été obtenus pour ce qui est des tests selon la norme SNV 195 921.

### Exemple 3

Dans un autre mode de réalisation, on a préparé une solution aqueuse à 5 % en PMEG (phosphate de méthylacrylate éthylène glycol). On a imprégné par foulardage exprimage des filés de fibres de viscose de Nm 50 présentés sous forme d'un tricot tubulaire de 140 g/m², préalablement blanchi avec un taux d'imprégnation de 75 %. Ce tricot a été irradié à 10 kGy, rincé et séché. Le taux de greffage obtenu est de 2 à 6 %. Le tricot est mis en contact avec une solution de carbonate de sodium à 5 %, puis rincé et séché. Il est ensuite mis en contact avec une solution aqueuse de PHMB à 5 % sous forme chlorydrate. Il est enfin rincé efficacement et séché. Un test microbiologique selon la norme SNV 195 920 donne comme résultats une cotation de 0 et une zone d'inhibition de 5 à 6 mm avec comme souche Staphylococcus aureus. Après cinq lavages en machine à laver à 40° C avec une solution de lessive ECE à 3 g/l, les mêmes tests ont été renouvelés et ont donné comme résultats une cotation 0 et une zone d'inhibition de 2 à 3 mm avec la souche Staphylococcus aureus et de 0 à 1 mm avec la souche Pseudomonas aeruginosa. Les mêmes résultats que ci-dessus ont été obtenus avec les tests selon la norme SNV 195 921.

### Exemple 4

Dans un autre exemple de réalisation, mettant en oeuvre également une solution aqueuse à 5 % en PMEG, on a imprégné un non-tissé composé à 80 % de viscose et 20 % de polyester et faisant 200 g/m² par foulardage exprimage avec un taux d'imprégnation de 75 %. Le non-tissé a été irradié à 10 kGy, rincé et séché. Le taux de greffage obtenu est de 2 à 6 %. Après avoir été mis en contact avec une solution de carbonate de sodium à 5 %, le non-tissé est rincé et séché puis de nouveau mis en contact avec une solution de MIRAPOL®550 à 5 %, rincé efficacement et séché. Les tests microbiologiques selon la norme SNV 195 920 avec la souche Staphylococcus aureus ont donné une cotation 0 et une zone d'inhibition de 2 à 4 mm. Après cinq lavages en machine à laver à 40° C avec une solution de lessive ECE à 3 g/l, la cotation est toujours de 0 et la zone d'inhibition est passée à 0 à 1 mm. Selon la norme SNV 195 921, les résultats sont strictement les mêmes que précédemment.

### Exemple 5

Dans un autre exemple de réalisation, on est parti d'une solution aqueuse à 10 % en acide acrylique dont on a imprégné des fibres de viscose présentées sous forme d'un tricot tubulaire de 140 g/m², par foulardage exprimage avec un taux d'imprégnation de 75 %. Les fibres sont irradiées à 10 kGy, rincées et séchées. Le taux de greffage obtenu est de 2 à 7 %. Le tricot est mis en contact avec une solution de carbonate de sodium à 5 %, rincé et séché. Une partie de ce tricot est mis en contact avec une solution de RHOQUAT®JL à 5 % dans l'eau, rincé efficacement et séché avant de subir les tests microbiologiques selon les normes SNV 195 920 et SNV 195 921. L'autre partie du tricot est mise en contact avec une solution de RHOQUAT® JL à 2 % et de PHMB sous forme de chlorydrate à 2 % dans l'eau, rincé efficacement et séché avant de subir les mêmes tests microbiologiques. S'agissant des tests sur la norme SNV 195 920 avec la souche Staphylococcus aureus, avant entretien lessiviel, dans les deux cas, la cotation était de 0 et la zone d'inhibition de 6 à 7 mm.

On a ensuite fait subir au premier tricot cinq lavages en machine à laver à 40° C avec une solution de lessive ECE à 3 g/l puis on l'a testé de nouveau avec la souche Staphylococcus aureus ; la cotation était de 0 et la zone d'inhibition de 1 à 2 mm tandis qu'avec la souche Pseudomonas aeroginosa la cotation était de 0 et la zone d'inhibition de 0 à 1 mm.

Quant au second tricot, il a subi cinq cycles de nettoyage à sec, conformément à la norme ISO 3 175 Cycle II "articles sensibles", avant d'être testé de nouveau selon la norme SNV 195 920, avec la souche Staphylococcus aureus la cotation était de 0 et la zone d'inhibition de 2 à 3mm et avec la souche Pseudomonas aeroginosa la cotation était de 0 et la zone d'inhibition de 1 à 2 mm.

Quant aux tests microbiologiques selon la norme SNV 195 921, la cotation a toujours été de 0 et les zones d'inhibition était respectivement avec la souche Candida albicans de 1 à 2 mm pour le premier tricot et de 2 à 3 mm pour le second et avec les souches Aspergillus niger et Tricophyton interdigitale de 0 à 1 mm pour les deux tricots.

### Exemple 6

Dans un dernier exemple de réalisation, on a préparé une solution aqueuse à 10 % en AMPS. On a imprégné une mousse à pores ouverts en polyuréthane de 50 g/m², de dureté Shore 10, avec un taux d'imprégnation, après exprimage, de 75 %. La mousse a été irradiée à 10 kGy puis rincée et séchée. Le taux de greffage obtenu était de l'ordre de 10 %. La mousse a été mise en contact avec une solution de carbonate de sodium à 5 %, rincée et séchée. Elle a ensuite été mise en contact avec une solution aqueuse de RHOQUAT®JL à 2 % de PHMB sous forme de chlorydrate à 2 % et de MIRAPOL®A 15 à 2 %, rincée efficacement et séchée.

Le test microbiologique selon la norme SNV 195 920 a donné, avec la souche Staphylococcus aureus une cotation 0 et une zone d'inhibition de 6 à 7 mm. Après cinq lavages en machine à laver à 40° C avec une solution de lessive ECE à 3 g/l, la mousse a été de nouveau testée selon cette même norme avec la souche Staphylococcus aureus, la cotation était de 0 et la zone d'inhibition de 2 à 3 mm et avec la souche Pseudomonas aeroginosa, la cotation était de 0 et la zone d'inhibition de 1 à 2 mm. Selon la norme SNV 195 921, avec les souches Candida albicans, Aspergillus niger et Tricophyton interdigitale; la cotation était de 0 et la zone d'inhibition respectivement de 2 à 3 mm pour la première souche et de 0 à 1 mm pour les deux autres souches.

### Exemple 7

On a préparé une solution aqueuse à 5% de dodécylsulfate de sodium (biocide) et une solution aqueuse à 5% d'ammonium quaternaire BAC®50% (biocide fabriqué par la société THOR). On a imprégné par foulardage un tissu type toile (140g/m²) à l'aide de la solution de dodécylsulfate de sodium, avec un taux d'imprégnation de 70%. Après séchage, le tissu est ensuite imprégné par pulvérisation à l'aide de la solution d'ammonium quaternaire. Le tissu est rincé à l'eau et séché.

Un test microbiologique selon SNV 195 920 avec la souche Staphylococcus aureus a donné une cotation O et une zone d'inhibition de 3mm. Après cinq lavages à 40°C, avec une solution de lessive ECE à 3g/l, la cotation est de O avec une zone d'inhibition de O à 1 mm.

### Exemple 8

On a préparé une solution aqueuse à 5% de dodécylsulfate de sodium (biocide) et une solution aqueuse à 5% de triméthylphényl ammonium bromure (utilisé comme antistatique ou tensio-actif). On a imprégné par foulardage un tissu type toile (140g/m²) à l'aide de la solution de dodécylsulfate de sodium, avec un taux d'imprégnation de 70%. Après séchage, le tissu est ensuite imprégné par pulvérisation à l'aide de la solution d'ammonium quaternaire. Le tissu est rincé à l'eau et séché.

Un test microbiologique selon SNV 195 920 avec la souche Staphylococcus aureus a donné une cotation O et une zone d'inhibition de 2 mm. Après cinq lavages à 40°C, avec une solution de lessive ECE à 3g/l, la cotation est de O sans zone d'inhibition.

### Exemple 9

On a préparé une solution aqueuse à 5% d'oléate de sodium (constituant non biocide de savon et de lessive) et une solution aqueuse à 5% d'ammonium quaternaire BAC®5O% (biocide fabriqué par la société THOR). On a imprégné par foulardage un tissu type toile (140 g/m²) à l'aide de la solution d'oléate de sodium, avec un taux d'imprégnation de 70%. Après séchage, le tissu est ensuite imprégné par pulvérisation à l'aide de la solution d'ammonium quaternaire. Le tissu est rincé à l'eau et séché.

Un test microbiologique selon SNV 195 920 avec la souche Staphylococcus aureus a donné une cotation O et une zone d'inhibition de 2mm. Après cinq lavages à 40°C, avec une solution de lessive ECE à 3g/l, la cotation est de O sans zone d'inhibition.

### Exemple 1O

On a préparé une solution aqueuse à 5% de polyacrylate de sodium (constituant non biocide de tensio-actifs anioniques) et une solution aqueuse à 5% d'ammonium quaternaire BAC®50% (biocide fabriqué par la société THOR). On a imprégné par foulardage un tissu type toile (140 g/m²) à l'aide de la solution de polyacrylate de sodium, avec un taux d'imprégnation de 70%. Après séchage, le tissu est ensuite imprégné par pulvérisation à l'aide de la solution d'ammonium quaternaire. Le tissu est rincé à l'eau et séché.

Un test microbiologique selon SNV 195 920 avec la souche Staphylococcus aureus a donné une cotation O et une zone d'inhibition de 2mm. Après cinq lavages à 40°C, avec une solution de lessive ECE à 3g/l, la cotation est de O sans zone d'inhibition.

### Exemple 11

On a préparé un tissu type toile (140g/m²) greffé par l'acide acrylique à un taux de 5% (selon le procédé décrit dans le premier exemple de réalisation). On a préparé une solution aqueuse à 5% d'ammonium quaternaire BAC®50% (biocide fabriqué par la société THOR). On imprègne le tissu greffé par l'acide acrylique par foulardage à l'aide de la solution d'ammonium quaternaire, avec un taux d'imprégnation de 75%. Le tissu est rincé à l'eau et séché.

Un test microbiologique selon SNV 195 920 avec la souche Staphylococcus aureus a donné une cotation O et une zone d'inhibition de 2mm. Après cinq lavages à 40°C, avec une solution de lessive ECE à 3g/l, la cotation est de O sans zone d'inhibition.

### Exemple 12

On a préparé un tissu type toile (140g/m²) greffé par un acrylate d'ammonium quaternaire (un acrylate porteur d'une chaîne en C18, avec n=2 et X=bromure) à un taux de 5% (selon le procédé décrit page 12). On a préparé une solution aqueuse à 5% de dodécylsulfate de sodium. On imprègne le tissu greffé d'ammonium quaternaire par foulardage à l'aide de la solution d'oléate de sodium, avec un taux d'imprégnation de 70%. Le tissu est rincé à l'eau et séché.

Un test microbiologique selon SNV 195 920 avec la souche Staphylococcus aureus a donné une cotation O et une zone d'inhibition de 6 mm. Après cinq lavages à 40°C, avec une solution de lessive ECE à 3g/l, la cotation est de O avec une zone d'inhibition de 3mm.

### Exemple 13

On a préparé un tricot tubulaire en coton - polyester 50% 50% (140g/m²) greffé par un acrylate d'ammonium quaternaire non biocide (PLEXIMO® ou méthacrylate d'éthyle triméthylammonium) à un taux de 5% (selon le procédé décrit dans le premier exemple de réalisation). On a préparé une solution aqueuse à 5% de dodécylsulfate de sodium. On imprègne le tricot tubulaire greffé d'ammonium quaternaire par foulardage à l'aide de la solution de dodécylsulfate de sodium, avec un taux d'imprégnation de 75%. Le tissu est rincé à l'eau et séché.

Un test microbiologique selon SNV 195 920 avec la souche Staphylococcus aureus a donné une cotation O et une zone d'inhibition de 5 mm. Après cinq lavages à 40°C, avec une solution de lessive ECE à 3g/l, la cotation est de O avec une zone d'inhibition de 1 mm.

### Exemple 14

On a préparé un tissu type toile (100 g/m²) greffé par un acrylate d'ammonium quaternaire (un acrylate porteur d'une chaîne en C18, avec n = 2 et X=bromure) à un taux de 5% (selon le procédé décrit page 12). Le contre-ion naturel est donc l'ion bromure. Le tissu est testé tel quel (après rinçage) selon SNV 195 920, avec la souche Staphylococcus aureus. On obtient une cotation O et une zone d'inhibition de 5mm.

On a préparé une solution aqueuse à 5% d'oléate de sodium (constituant non biocide de savon et de lessive). On imprègne le tissu greffé d'ammonium quaternaire par foulardage à l'aide de la solution d'oléate de sodium, avec un taux d'imprégnation de 70%. Le tissu est rincé à l'eau et séché. Le contre-ion bromure est remplacé par l'anion oléate. Ce type d'échange a lieu naturellement lors d'un savonnage ou d'une lessive. Le tissu est rincé à l'eau et séché.

Un test microbiologique selon SNV 195 920 avec la souche Staphylococcus aureus a donné une cotation O et une zone d'inhibition de 4mm. Après cinq lavages à 40°C, avec une solution de lessive ECE à 3g/l, la cotation est de O avec une zone d'inhibition de 1mm.

### Exemple 15

On a préparé un tricot tubulaire en coton - polyamide 40% 60% (120g/m²) greffé par un acrylate d'ammonium quaternaire (un mélange équimolaire d'acrylates C14, C16 et C18, avec n = 2 et X = bromure) à un taux de 5% (selon le procédé décrit page 12). Le contre-ion naturel est donc l'ion bromure. Le tricot est testé tel quel (après rinçage) selon SNV 195 920, avec la souche Staphylococcus aureus. On obtient une cotation O et une zone d'inhibition de 5mm.

On a préparé une solution aqueuse à 5% de polyacrylate de sodium (constituant non biocide de tensio-actifs anioniques). On imprègne le tricot greffé d'ammonium quaternaire par foulardage à l'aide de la solution de polyacrylate de sodium, avec un taux d'imprégnation de 70%. Le tricot tubulaire est rincé à l'eau et séché. Le contre-ion bromure est remplacé par le polyanion polyacrylate. Cet échange a pour but de protéger le greffon ammonium quaternaire pour des applications spécifiques. Le tricot tubulaire est rincé à l'eau et séché.

Un test microbiologique selon SNV 195 920 avec la souche Staphylococcus aureus a donné une cotation O et une zone d'inhibition de 2mm. Après cinq lavages à 40°C, avec une solution de lessive ECE à 3g/l, la cotation est de O avec une zone d'inhibition de 1 mm.

Le procédé de l'invention n'est pas limité aux exemples de réalisation décrits ci-desssus, donnés à titre non exhausitf. En particulier, s'agissant de l'immobilisation in situ du polymère par insolubilisation grâce à un anion à haut poids moléculaire le rendant insoluble ou peu soluble, on peut utiliser comme anion à haut poids moléculaire un polymère anionique, notamment dans le cas d'un polymère cationique à insolubiliser, par exemple du type polyacrylate ou polyméthacrylate, qui peut être obtenu par polymérisation de monomères tels que l'acide acrylique, méthacrylique, AMPS, PMEG.

De plus, s'agissant de l'immobilisation in situ d'un polymère cationique sur un greffon fixé sur le matériau fibreux ou poreux, on peut procéder en deux phases. Dans une première phase, on met en oeuvre une solution dans laquelle est dissous un anion à haut poids moléculaire en présence du polymère cationique antiseptique, mais l'anion étant dans une concentration par défaut telle que le sel obtenu est soluble.

Dans une seconde phase, la solution contenant ce sel imprègne le matériau fibreux ou poreux greffé dont les greffons sont porteurs de fonctions anioniques. On obtient dans ce cas une immobilisation in situ d'une plus grande quantité de polymère cationique antiseptique.

De plus, on peut utiliser les polymères ammonium quaternaire issus de la polymériation de monomères du type acrylate ou méthacrylate porteurs de fonction antiabactérienne ammonium quaternaire, tels que visés précédemment.

Les domaines d'application du matériau fibreux ou poreux, présentant des propriétés antiseptiques, obtenu grâce au procédé de l'invention sont multiples. Un domaine privilégié est celui des sous-vêtements et des articles chaussants qui, en raison de leur utilisation en contact direct avec la peau, sont un lieu privilégié pour la fixation, l'adhérence et le développement des micro-organismes. Certes, la plupart des micro-organismes présents sur la peau ne sont pas pathogènes et ne doivent pas être combattus. Cependant, certaines souches peuvent être responsables de mycoses, par exemple Tricophyton interdigitale responsable du phénomène dénommé "pied d'athlète" et qui occasionne des rougeurs et de fortes démangeaisons entre les orteils.

Du fait de l'absorption de la sueur, le matériau textile est le siège d'un développement de micro-organismes variés, dont on a montré qu'ils étaient en phase de croissance exponentielle après un porter de 4 h, pouvant atteindre une concentration de 10⁷ germes par cm² de textile après 8 h. Cette concentration peut avoir comme conséquences d'une part la dégradation et la coloration des fibres naturelles, certains micro-organismes possédant des enzymes capables de dégrader les fibres comme la cellulose, la laine ou le lin et d'autre part des mauvaises odeurs.

Le procédé de traitement de l'invention permet de lutter efficacement contre ces inconvénients.

Un autre domaine d'application concerne les mousses et textiles à usage hospitalier, notamment les champs opératoires, draps de lits et blouses. Un autre domaine d'application est celui des tapis, moquettes, revêtements muraux, filtres à air ou à liquide des secteurs industriels, hospitaliers ou grand public.

## Revendications

1. Procédé de traitement d'un matériau en vue de lui conférer des propriétés biocides et/ou biostatiques d'une très grande stabilité à l'entretien, procédé selon lequel on met en oeuvre une solution de traitement dans laquelle est dissoute au moins une molécule ionique et/ou au moins un polymère ionique et qui comprend une ou plusieurs étapes au cours de laquelle ou desquelles d'une part on applique ladite solution de traitement et d'autre part la molécule ionique et/ou ledit polymère ionique réagit avec un agent précipitant pour former , in situ, sur ledit matériau un précipité présentant des propriétés biocides et/ou biostatiques , **caractérisé en ce que**, ladite molécule et/ou ledit polymère et/ou l'agent précipitant étant biocide(s) et/ou biostatique(s), ledit précipité est un sel qui est immobilisé et donc rendu insoluble dans l'eau, l'alcool, la soude, et les agents d'entretien comportant notamment des agents tensio-actifs anioniques alkylés , soit du fait de la présence dans ledit sel d'un anion de haut poids moléculaire sélectionné dans le groupe :
- monoanion de formule CₙH₂ₙ₊₁-A, n étant au moins 12,
- polyanion de formule 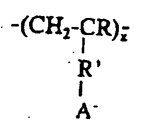 dans laquelle x est supérieur à 10, R est H ou CH3, R' est un groupe d'atomes,
- anion complexe minéral, de type silicate ou polyphosphate,
soit du fait de l'appartenance dudit sel à un greffon fixé sur ledit matériau.

2. Procédé selon la revendication 1, **caractérisé en ce que** , après l'application sur le matériau de la solution de traitement et son éventuel séchage, on utilise comme agent précipitant une solution ou une émulsion contenant au moins une molécule ionique et/ou au moins un polymère ionique en sorte d'obtenir un précipité insoluble.

3. Procédé selon la revendication 2 , **caractérisé en ce que** ladite solution de traitement ou ledit agent précipitant contient une solution aqueuse contenant 5% de chlorure de benzalkonium et 5% de docécylsulfate de sodium.

4. Procédé selon la revendication 2 ou 3 , **caractérisé en ce que** la solution de traitement ou l'agent précipitant contient un polymère cationique antiseptique choisi parmi le groupe formé par les polymères polyhexaméthylène biguanides bactéricides (PHMB) et par les polymères ammonium quaternaire bactéricides.

5. Procédé selon l'une quelconque des revendications 2 à 4 , **caractérisé en ce que** la solution de traitement ou l'agent précipitant contient un anion silicate.

6. Procédé selon l'une quelconque des revendications 2 à 4, **caractérisé en ce que** l'agent de traitement ou l'agent précipitant contient un polymère anionique alkylé ayant au moins dix atomes de carbone, par exemple un savon de sodium de C12 à C22 , notamment oléate de sodium , alkylbenzène sulfonate de sodium ou tripolyphosphate de sodium.

7. Procédé selon l'une quelconque des revendications 2 à 4 , **caractérisé en ce que** la solution de traitement ou l'agent précipitant contient une solution de dodécyl sulfate de sodium.

8. Procédé selon la revendication 1 **caractérisé en ce qu'**il comporte une étape consistant à greffer sur le matériau des greffons porteurs de fonctions anioniques ou cationiques ayant la fonction d'agent précipitant et qui sont donc aptes à précipiter la molécule et/ou le polymère ionique dissout(e) dans la solution de traitement.

9. Procédé selon la revendication 8 , **caractérisé en ce que** les greffons sont des acrylates ou méthacrylates d'alkyle, porteurs de fonctions anioniques.

10. Procédé selon la revendication 8 , **caractérisé en ce que** le greffon comportant une fonction carboxylique ou carboxylate , on utilise comme monomère de greffage l'acide acrylique ou l'acide méthacrylique.

11. Procédé selon la revendication 8 , **caractérisé en ce que** le greffon comportant une fonction sulfonique ou sulfonate , on utilise comme monomère de greffage l'acide 2-acrylamido-2-méthylpropane sulfonique (AMPS).

12. Procédé selon la revendication 8, **caractérisé en ce que** le greffon comportant une fonction phosphorique ou phosphate , on utilise comme monomère de greffage le phosphate de méthylacrylate éthylène glycol (PMEG).

13. Procédé selon l'une quelconque des revendications 1 à 12 **caractérisé en ce que** ledit matériau est un matériau présentant une grande surface spécifique , par exemple un matériau fibreux ou poreux.

## Claims

1. A method of treating a material to endow it with biocidal and/or biostatic properties with very good stability to laundering, in which method a treatment solution is used in which at least one ionic molecule and/or at least one ionic polymer is dissolved and which comprises one or more steps during which, firstly, said treatment solution is applied and the ionic molecule and/or said ionic polymer reacts with a precipitating agent to form, in situ, on said material, a precipitate having biocidal and/or biostatic properties, **characterized in that** said molecule and/or said polymer and/or the precipitating agent is/are biocidal and/or biostatic, said precipitate being a salt which is immobilized and thus rendered insoluble in water, alcohol, sodium hydroxide, and laundering agents particularly comprising alkylated anionic surfactants, either because of the presence in said salt of a high molecular weight anion selected from the following group:
·monoanion which formula CₙH₂ₙ₊₁-A⁻, in which n is at least 12;
. a polyanion with formula: 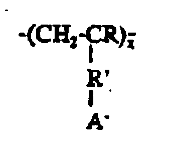 in which x is more than 10, R is H or CH₃, R' is a group of atoms;
a complex mineral anion of the silicate or polyphosphate type;
or because said salt belongs to a graft fixed on said material.

2. A method according to claim 1, **characterized in that**, after application of the treatment solution to the material and its optional drying, a solution or emulsion containing at least one ionic molecule and/or at least one ionic polymer is used as the precipitating agent to obtain an insoluble precipitate.

3. A method according to claim 2, **characterized in that** said treatment solution or said precipitating agent contains an aqueous solution containing 5% of benzalkonium chloride and 5% of sodium dodecylsulfate.

4. A method according to claim 2 or claim 3, **characterized in that** the treatment solution or precipitating agent contains an antiseptic cationic polymer selected from the group formed by bactericidal polyhexamethylene biguanide (PHMB) polymers and bactericidal quaternary ammonium polymers.

5. A method according to any one of claims 2 to 4, **characterized in that** the treatment solution or the precipitating agent contains a silicate anion.

6. A method according to any one of claims 2 to 4, **characterized in that** the treatment agent or precipitating agent contains an alkylated anionic polymer containing at least ten carbon atoms, for example a C12 to C22 sodium soap, in particular sodium oleate, sodium alkylbenzene sulfonate or sodium tripolyphosphate.

7. A method according to any one of claims 2 to 4, **characterized in that** the treatment solution or the precipitating agent contains a solution of sodium dodecylsulfate.

8. A method according to claim 8, **characterized in that** it comprises a step consisting in grafting onto the material grafts carrying anionic or cationic functions acting as a precipitating agent and which are thus capable of precipitating the ionic polymer and/or molecule dissolved in the treatment solution.

9. A method according to claim 8, **characterized in that** the grafts are alkyl acrylates or methacrylates, carrying anionic functions.

10. A method according to claim 8, **characterized in that** when the graft comprises a carboxylic or carboxylate function, the grafting monomer used is acrylic acid or methacrylic acid.

11. A method according to claim 8, **characterized in that** when the graft comprises a sulfonic or sulfonate function, the grafting monomer used is 2-acrylamido-2-methylpropanesulfonic acid (AMPS).

12. A method according to claim 8, **characterized in that** when the graft comprises a phosphoric or phosphate function, the grafting monomer used is ethylene glycol methacrylate phosphate (EGMP).

13. A method according to any one of claims 1 to 12, **characterized in that** said material is a material having a large specific surface area, for example a fibrous or porous material.

## Patentansprüche

1. Verfahren zur Behandlung eines Materials im Hinblick darauf, ihm biozide und/oder biostatische Eigenschaften mit einer sehr großen Stabilität bei der Reinigung zu verleihen, gemäß welchem Verfahren man eine Behandlungslösung einsetzt, in der wenigstens ein ionisches Molekül und/oder wenigstens ein ionisches Polymer gelöst ist und welches eine oder mehrere Stufen umfaßt, bei der oder denen man einerseits die Behandlungslösung anwendet und andererseits das ionische Molekül und/oder das ionische Polymer mit einem Fällungsmittel reagiert, um in situ auf dem Material einen Niederschlag zu bilden, der biozide und/oder biostatische Eigenschaften aufweist, **dadurch gekennzeichnet, daß** das Molekül und/oder das Polymer und/oder das Fällungsmittel biozid und/oder biostatisch ist (sind), der Niederschlag ein Salz ist, welches immobilisiert ist und daher unlöslich gemacht in Wasser, Alkohol, Soda und Pflegemitteln, die insbesondere anionische Alkyltenside umfassen entweder aufgrund der Gegenwart in dem Salz von einem Anion mit hohem Molekulargewicht, das gewählt ist aus der Gruppe:
- Monoanion mit Formel CₙH₂ₙ₊₁-A⁻, wobei n wenigstens 12 ist,
- Polyanion mit Formel 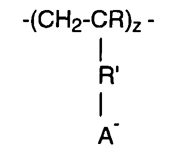 in welcher x größer als 10 ist, R H oder CH3 ist, R' eine Atomgruppe ist,
- komplexes mineralisches Anion vom Silikat- oder Polyphosphattyp,
oder aufgrund der Zugehörigkeit des Salzes zu einer Pfropfung, die auf dem Material befestigt ist.

2. Verfahren nach Anspruch 1, **dadurch gekennzeichnet, daß** nach Auftragen auf das Material von der Behandlungslösung und seinem eventuellen Trocknen man als Fällungsmittel eine Lösung oder Emulsion verwendet, die wenigstens ein ionisches Molekül und/oder wenigstens ein ionisches Polymer enthält, derart, daß ein unlöslicher Niederschlag erhalten wird.

3. Verfahren nach Anspruch 2, **dadurch gekennzeichnet, daß** die Behandlungslösung oder das Fällungsmittel eine wäßrige Lösung enthält, die 5% Benzylalkoniumchlorid und 5% Natriumdodecylsulfat enthält.

4. Verfahren nach Anspruch 2 oder 3, **dadurch gekennzeichnet, daß** die Behandlungslösung oder das Fällungsmittel ein kationisches antiseptisches Polymer enthält, gewählt aus der Gruppe, die gebildet wird durch die Bakteriziden Polyhexamethylen-Biguanid-Polymere (PHMB) und die bakteriziden quaternären Ammonium-Polymere.

5. Verfahren nach einem der Ansprüche 2 bis 4, **dadurch gekennzeichnet, daß** die Behandlungslösung oder das Fällungsmittel ein Silikatanion enthält.

6. Verfahren nach einem der Ansprüche 2 bis 4, **dadurch gekennzeichnet, daß** das Behandlungsmittel oder das Fällungsmittel ein alkyliertes anionisches Polymer mit wenigstens 10 Kohlenstoffatomen enthält, zum Beispiel eine Natriumseife mit C12 bis C22, insbesondere Natriumoleat, Natriumalkylbenzolsulfonat oder Natriumtripolyphosphat.

7. Verfahren nach einem der Ansprüche 2 bis 4, **dadurch gekennzeichnet, daß** die Behandlungslösung oder das Fällungsmittel eine Natriumdodecylsulfatlösung enthält.

8. Verfahren nach Anspruch 1, **dadurch gekennzeichnet, daß** es eine Stufe umfaßt, die darin besteht, auf dem Material Pfropfungen aufzupfropfen, die Träger von anionischen oder kationischen Funktionen mit der Funktion eines Fällungsmittels sind und die daher geeignet sind, das Molekül und/oder das ionische Polymer zu fällen, das in der Behandlungslösung gelöst ist.

9. Verfahren nach Anspruch 8, **dadurch gekennzeichnet, daß** die Pfropfungen Alkylacrylate oder Alkylmethacrylate sind, die Träger von anionischen Funktionen sind.

10. Verfahren nach Anspruch 8, **dadurch gekennzeichnet, daß** die Pfropfung eine Carboxyl- oder Carboxylatfunktion umfaßt, man als Pfropfmonomer Acrylsäure oder Metacrylsäure verwendet.

11. Verfahren nach Anspruch 8, **dadurch gekennzeichnet, daß** die Pfropfung eine Sulfon- oder Sulfonatfunktion umfaßt, man als Pfropfmonomer 2-Acrylamido-2-Methylpropansulfonsäure (AMPS) verwendet.

12. Verfahren nach Anspruch 8, **dadurch gekennzeichnet, daß** die Pfropfung eine phosphorhaltige Funktion oder Phosphatfunktion umfaßt und man als Pfropfmonomer Ethylenglycol-Methacrylatphosphat (PMEG) verwendet.

13. Verfahren nach einem der Ansprüche 1 bis 12, **dadurch gekennzeichnet, daß** das Material ein Material ist, das eine große spezifische Oberfläche aufweist, zum Beispiel ein faseriges oder poröses Material.
